(19) Europäisches Patentamt
European Patent Office
Office européen des brevets

(11) Publication number: **0 104 888**
**B1**

(12) **EUROPEAN PATENT SPECIFICATION**

(45) Date of publication of patent specification: **15.10.86**

(21) Application number: **83305613.8**

(22) Date of filing: **22.09.83**

(51) Int. Cl.[4]: **C 07 C 89/02**, C 07 C 91/16, C 07 C 93/14, C 07 C 103/28, C 07 F 7/18

(54) **Process for preparing 2-hydroxy-2-phenylethylamines.**

(30) Priority: **27.09.82 US 424785**

(43) Date of publication of application:
**04.04.84 Bulletin 84/14**

(45) Publication of the grant of the patent:
**15.10.86 Bulletin 86/42**

(84) Designated Contracting States:
**BE CH DE FR GB IT LI LU NL SE**

(56) References cited:
**EP-A-0 007 204**

**CHEMISCHE BERICHTE, VOL. 102, 1969, PP. 14-22, VERLAG CHEMIE GMBH, WEINHEIM (DE); L.BIRKOFER ET AL.: "REAKTION N-SILYLIERTER SEKUNDäRER CARBONSäUREAMIDE MIT EPOXIDEN UND KETONEN"**

**CHEMICAL ABSTRACTS, vol. 78, no. 17, 30th April 1973, P. 387, NO. 110062Q, COLUMBUS, OHIO (US); P.K.POOK: "STRUCTURE IDENTIFICATION OF CATECHOL AMINE-TYPE COMPOUNDS BY GAS CHROMATOGRAPHY-MASS SPECTROMETRY OF THEIR SILYL, ACYL AND KETONE DERIVATIVES"**

(73) Proprietor: **ELI LILLY AND COMPANY**
**307, East McCarty Street**
**Indianapolis Indiana 46285 (US)**

(72) Inventor: **Weigel, Leland Otto**
**241, West Hampton Drive**
**Indianapolis Indiana 46208 (US)**

(74) Representative: **Crowther, Terence Roger et al**
**Erl Wood Manor**
**Windlesham Surrey GU20 6PH (GB)**

(56) References cited:
**CHEMICAL ABSTRACTS, VOL. 73, NO. 13, 28 SEP 1970, P. 262, NO. 65591V, COLUMBUS, OHIO (US); J.E.FORREST ET AL.: NUCLEAR MAGNETIC RESONANCE SPECTROSCOPY OF THE TRIMETHYLSILYL ETHERS OF SOME HYDROXYPHENYLALKYLAMINES"**

**EP 0 104 888 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention belongs to the field of pharmaceutical and synthetic organic chemistry, and provides a process for preparing 2-hydroxy-2-phenylethylamines by the direct reaction of a styrene oxide, which may be substituted on the phenyl ring, with the silylated derivative of the desired amine. The products of the process include a wide variety of hydroxy-substituted amines useful in many fields of pharmaceutical chemistry. The process is highly selective in its ability to break the epoxide moiety of the styrene oxide, and to provide for attack of the amine at the β-carbon, rather than at the α-carbon.

Compounds of the general type produced by the process of this invention have long been of interest to pharmaceutical chemists, and many processes for making them have been studied. European Patent Application No. 79301282.4 describes the preparation of this type of compound by the direct coupling of styrene oxide, or a ring-substituted styrene oxide, with the appropriate phenylalkylamine, at 50—80°.

Various workers in the past have carried out processes which open epoxides to attack by active substituting agents. For example, Guindon et al., *Syn. Comm. 11*, 391—98 (1981), used aryl- or alkylthiotrialkylsilanes to substitute on styrene oxide. When they used zinc iodide as a catalyst in the reaction, they obtained primarily attack at the carbon α to the phenyl ring, but, when butyllithium was used as catalyst, they found primarily attack at the β-carbon.

Andrews et al., *Tet. Let. 22*, 3803—06 (1981), reacted styrene oxide with monohalosilanes, and found that a catalyst was usually necessary. Their most effective catalysts were triphenylphosphine and tetraalkylammonium chlorides. They obtained high yields of products having a silylated hydroxy group at the α-carbon and a halogen atom at the β-carbon.

Olah et al., *Synthesis*, 280—81 (1981), opened epoxide rings with a perfluorinated resin acid catalyst called Nafion-H. When the process was carried out in an alcohol, they obtained primarily the product wherein an alkoxy group was at the α-carbon position, and a hydroxy at the β-carbon position.

Some trimethylsilyl ethers of certain hydroxyphenylalkylamines are described by Birkofer et al, Chem. Ber. 1969, 102(1), 14—22, by Poole et al, Proc. Int. Symp. Gas Chromatography Mass Spectron. 1972, 177—87 and by Forrest et al, J. Pharm. Pharmacol., 22(7) 512—17.

The present invention provides a process for preparing an amine of the formula

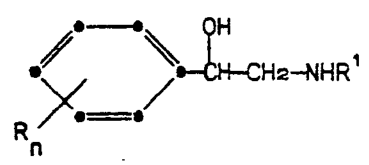

or an acid addition salt thereof; wherein

n is 0, 1 or 2;

the R groups are the same or different, when n is 2, and are chosen from chloro, bromo, fluoro, nitro, $C_1$—$C_4$ alkyl, $C_1$—$C_4$ alkoxy, $C_1$—$C_4$ alkylsulfonyl, hydroxy, carboxy, amino, aminocarbonyl, mono- or di($C_1$—$C_4$ alkyl)amino, or mono- or di($C_1$—$C_4$ alkyl)aminocarbonyl; provided that when n is 2, the R groups are not both *ortho*; provided that an alkoxy R group is not *para;*

$R^1$ is $C_3$—$C_8$ cycloalkyl, or $C_3$—$C_8$ cycloalkyl mono- or disubstituted with $C_1$—$C_4$ alkyl, $C_1$—$C_4$ alkoxy, chloro, bromo or fluoro,

$C_1$—$C_6$ alkyl,

$C_1$—$C_6$ alkyl substituted with chloro, bromo or fluoro,

$C_1$—$C_6$ alkyl monosubstituted with $C_1$—$C_4$ alkoxy, $C_1$—$C_4$ alkanoyl, $C_1$—$C_4$ alkoxycarbonyl, hydroxy, amino, phenyl, or phenyl mono- or disubstituted with chloro, bromo, fluoro, nitro, cyano, $C_1$—$C_4$ alkyl, $C_1$—$C_4$ alkoxy, $C_1$—$C_4$ alkanoyl, $C_1$—$C_4$ alkoxycarbonyl, hydroxy, carboxy, amino, aminocarbonyl, mono- or di($C_1$—$C_4$ alkyl)amino, or mono- or di($C_1$—$C_4$ alkyl)aminocarbonyl;

which process comprises reacting an epoxide of the formula

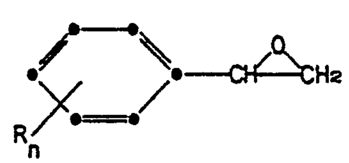

with a silylated amine of the formula

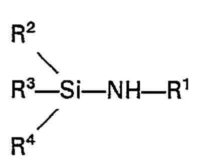

wherein

$R^2$ and $R^3$ are independently phenyl or $C_1$—$C_4$ alkyl, and $R^4$ is $C_1$—$C_4$ alkyl; provided that carboxy, hydroxy, unsubstitued amino and unsubstituted aminocarbonyl groups on the epoxide and the silylated amine are silylated with the group

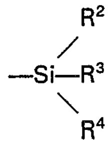 ;

in the presence of dimethylsulfoxide or without a solvent; to prepare a silylated intermediate of the formula

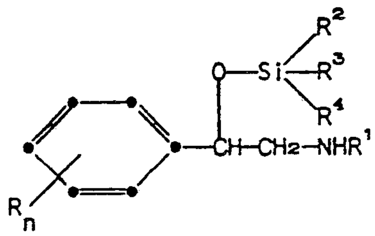

and hydrolyzing the silylated intermediate.

Silylated intermediates of the formula

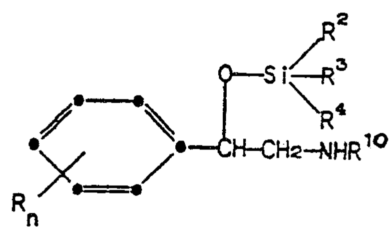

wherein

n is 0, 1 or 2;

the R groups are the same or different, when n is 2, and are chosen from chloro, bromo, fluoro, nitro, $C_1$—$C_4$ alkyl, $C_1$—$C_4$ alkoxy, $C_1$—$C_4$ alkylsulfonyl, hydroxy, carboxy, amino, aminocarbonyl, mono- or di($C_1$—$C_4$ alkyl)amino, or mono- or di($C_1$—$C_4$ alkyl)aminocarbonyl; provided that when n is 2, the R groups are not both *ortho*; provided that an alkoxy R group is not *para*;

$R^{10}$ is $C_3$—$C_8$ cycloalkyl, or $C_3$—$C_8$ cycloalkyl mono- or disubstituted with $C_1$—$C_4$ alkyl, $C_1$—$C_4$ alkoxy, chloro, bromo or fluoro,

$C_1$—$C_6$ alkyl substituted with chloro, bromo or fluoro,

$C_1$—$C_6$ alkyl monosubstituted with $C_1$—$C_4$ alkoxy, $C_1$—$C_4$ alkanoyl, $C_1$—$C_4$ alkoxycarbonyl, hydroxy, amino, phenyl, or phenyl mono- or disubstituted with chloro, bromo, fluoro, nitro, cyano, $C_1$—$C_4$ alkyl, $C_1$—$C_4$ alkoxy, $C_1$—$C_4$ alkanoyl, $C_1$—$C_4$ alkoxycarbonyl, hydroxy, carboxy, amino, aminocarbonyl, mono- or di($C_1$—$C_4$ alkyl)amino, or mono- or di($C_1$—$C_4$ alkyl)aminocarbonyl;

provided that when n is 0, $R^{10}$ is not $C_1$—$C_6$ alkyl monosubstituted with phenyl monosubstituted with hydroxy and provided that carboxy, hydroxy, unsubstituted amino and unsubstituted aminocarbonyl groups are silylated with the group

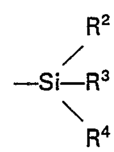

are also provided in one aspect of the invention.

In this document, all temperatures are expressed in degrees Celsius.

The general chemical terms used in the formula above carry their usual meanings in organic chemistry. For example, the terms $C_1$—$C_4$ alkyl, $C_1$—$C_4$ alkoxy, $C_1$—$C_4$ alkylsulfonyl, $C_1$—$C_4$ alkanoyl, $C_1$—$C_4$ alkoxycarbonyl, $C_1$—$C_6$ alkyl and $C_3$—$C_8$ cycloalkyl refer to such groups as methyl, ethyl, isopropyl, butyl, *s*-butyl, isobutyl, *t*-butyl, methoxy, propoxy, *s*-butoxy, *t*-butoxy, methylsulfonyl, ethylsulfonyl, isopropylsulfonyl, butylsulfonyl, isobutylsulfonyl, pentyl, hexyl, 1-methylbutyl, 1-ethylpropyl, 1-ethylbutyl, 2-ethylbutyl, formyl, acetyl, propionyl, butyryl, 2-methylpropionyl, methoxycarbonyl, ethoxycarbonyl, isopropoxycarbonyl, *t*-butoxycarbonyl, cyclopropyl, cyclobutyl, cyclopentyl, cyclohexyl, cycloheptyl, cyclooctyl and the like.

It is believed that the reader will easily understand the nature of the amines which are prepared by the process of this invention. The following group of exemplary products are mentioned, however, to assure understanding.

2-hydroxy-N-methyl-2-phenylethylamine
2-(3-chlorophenyl)-N-ethyl-2-hydroxyethylamine
2-(4-bromophenyl)-2-hydroxy-N-isopropylethylamine
N-butyl-2-(3-fluorophenyl)-2-hydroxyethylamine
N-*t*-butyl-2-hydroxy-2-(4-nitrophenyl)ethylamine
2-hydroxy-2-(2-methylphenyl)-N-pentylethylamine
2-(4-ethylphenyl)-2-hydroxy-N-(2,2-dimethylpropyl)ethylamine
2-hydroxy-2-(2-isopropylphenyl)-N-(1,1-dimethylpropyl)ethylamine
2-(4-butylphenyl)-2-hydroxy-N-(2-methylbutyl)ethylamine
N-hexyl-2-hydroxy-2-(3-isobutylphenyl)ethylamine
2-hydroxy-2-(3-methoxyphenyl)-N-(1,2-dimethylbutyl)ethylamine
2-(2-ethoxyphenyl)-N-(1-ethylbutyl)-2-hydroxyethylamine
N-chloromethyl-2-hydroxy-2-(3-propoxyphenyl)ethylamine
2-(2-*s*-butoxyphenyl)-N-(2-bromoethyl)-2-hydroxyethylamine
N-(1-fluoroethyl)-2-(3-*t*-butoxyphenyl)-2-hydroxyethylamine
N-(3,3,3-trifluoropropyl)-2-hydroxy-2-(4-methylsulfonylphenyl)ethylamine
N-(3,4-dichlorobutyl)-2-(3-ethylsulfonylphenyl)-2-hydroxyethylamine
2-(2-butylsulfonylphenyl)-N-(2-fluoro-1-methylpropyl)-2-hydroxyethylamine
2-(4-*s*-butylsulfonylphenyl)-N-(chloro-*t*-butyl)-2-hydroxyethylamine
N-(3-bromo-1-methylbutyl)-2-hydroxy-2-(3-hydroxyphenyl)ethylamine
2-(2-carboxyphenyl)-2-hydroxy-N-(2-methyl-2-trifluoromethylpropyl)ethylamine
2-(3-aminophenyl)-N-(1,2-dichlorohexyl)-2-hydroxyethylamine
2-(4-aminocarbonylphenyl)-N-heptachloropropyl-2-hydroxyethylamine
2-hydroxy-N-ethoxymethyl-2-(4-dimethylaminocarbonylphenyl)ethylamine
2-(2-ethylaminocarbonylphenyl)-2-hydroxy-N-(2-methoxyethyl)ethylamine
2-hydroxy-2-(3-propylaminocarbonylphenyl)-N-(3-propoxybutyl)ethylamine
2-(4-dibutylaminocarbonylphenyl)-N-(2-isobutoxyhexyl)-2-hydroxyethylamine.
2-(3-isobutylaminocarbonylphenyl)-N-cyclopropyl-2-hydroxyethylamine
N-cyclobutyl-2-hydroxy-2-(3-isopropylmethylaminocarbonylphenyl)ethylamine
N-cyclopentyl-2-(2-ethylpropylaminocarbonylphenyl)-2-hydroxyethylamine
N-cycloheptyl-2-(3-dimethylaminophenyl)-2-hydroxyethylamine
N-cyclooctyl-2-hydroxy-2-(4-propylaminophenyl)ethylamine
2-(3-diisobutylaminophenyl)-2-hydroxy-N-(2-methylcyclopropyl)ethylamine
2-(3-*t*-butylaminophenyl)-N-(2-chloro-3-ethylcyclobutyl)-2-hydroxyethylamine
2-(2-butylmethylaminophenyl)-2-hydroxy-N-(3-isopropylcyclopentyl)ethylamine
N-(1-isobutyl-3-fluorocyclopentyl)-2-(4-*s*-butylethylaminophenyl)-2-hydroxyethylamine
N-(2,4-dichlorocyclohexyl)-2-(3-diethylaminophenyl)-2-hydroxyethylamine
N-(3,5-dibromocyclooctyl)-2-(2,5-dichlorophenyl)-2-hydroxyethylamine
2-(3,4-difluorophenyl)-N-(3-fluoro-4-methoxycyclohexyl)-2-hydroxyethylamine
N-(5-ethyl-2-propoxycyclopentyl)-2-hydroxy-2-(2,4-dinitrophenyl)ethylamine
N-(4-butylcyclohexyl)-2-hydroxy-2-(3,5-dimethylphenyl)ethylamine
2-(2,4-dibutylphenyl)-N-(3-*t*-butoxycycloheptyl)-2-hydroxyethylamine
2-(2,3-diisobutylphenyl)-N-(2-formylethyl)-2-hydroxyethylamine
N-(acetylmethyl)-2-hydroxy-2-(3,5-dimethoxyphenyl)ethylamine
2-(2,5-diethoxyphenyl)-2-hydroxy-N-(4-propionylhexyl)ethylamine
2-(2,3-dibutoxyphenyl)-N-(2-butyrylbutyl)-2-hydroxyethylamine
2-[3,5-bis(*s*-butoxy)phenyl]-2-hydroxy-N-[3-(2-methylpropionyl)-1-methylbutyl]ethylamine
2-hydroxy-N-(6-methoxycarbonylhexyl)-2-(2,4-dimethylsulfonylphenyl)ethylamine
N-(4-ethoxycarbonylbutyl)-2-hydroxy-2-(2,5-diisopropylsulfonylphenyl)ethylamine
2-(3,5-diisobutylsulfonylphenyl)-2-hydroxy-N-(2-methyl-3-propoxycarbonylbutyl)ethylamine
N-(3-butoxycarbonyl-1-ethylpropyl)-2-[3,4-bis(*t*-butylsulfonyl)phenyl]-2-hydroxyethylamine
2-(2,5-dihydroxyphenyl)-2-hydroxy-N-(isopropoxycarbonylmethyl)ethylamine
2-(3,5-dicarboxyphenyl)-2-hydroxy-N-(hydroxymethyl)ethylamine
2-(2,4-diaminophenyl)-N-(2-hydroxyisopropyl)-2-hydroxyethylamine
2-(2,4-diaminocarbonylphenyl)-2-(hydroxy-*t*-butyl)-2-hydroxyethylamine
2-[2,5-di(ethylamino)phenyl]-N-(3-hydroxy-1-methylbutyl)-2-hydroxyethylamine
2-[3,5-bis(dimethylamino)phenyl]-N-(6-hydroxyhexyl)-2-hydroxyethylamine
N-aminomethyl-2-hydroxy-2-[2,4-bis(methylpropylamino)phenyl]ethylamine
N-(2-aminoethyl)-2-[3,4-bis(*s*-butylamino)phenyl]-2-hydroxyethylamine

2-[2,5-bis(isobutylamino)phenyl]-2-hydroxy-N-(2-aminopropyl)ethylamine
N-(4-aminoisobutyl)-2-hydroxy-2-[2,4-bis(butylmethylamino)phenyl]ethylamine
N-(3-amino-1,1-dimethylpropyl)-2-hydroxy-2-[3,4-bis(dimethylaminocarbonyl)phenyl]ethylamine
N-(3-amino-2-ethylbutyl)-2-[3,5-bis(ethylaminocarbonyl)phenyl]-2-hydroxyethylamine
N-benzyl-2-hydroxy-2-[2,4-bis(propylaminocarbonyl)phenyl]ethylamine
2-[2,5-bis(ethylisobutylaminocarbonyl)phenyl]-2-hydroxy-N-(2-phenylisopropyl)ethylamine
2-[3,4-bis(*t*-butylaminocarbonyl)phenyl]-2-hydroxy-N-(1,1-dimethylbutyl)ethylamine
2-hydroxy-2-[2,4-bis(methylaminocarbonyl)phenyl]-N-(3-phenylbutyl)ethylamine
2-(5-bromo-2-chlorophenyl)-2-hydroxy-N-(1-methyl-3-phenylpropyl)ethylamine
2-(2-bromo-4-hydroxyphenyl)-N-(3-chlorobenzyl)-2-hydroxyethylamine
N-[6-(2-bromophenyl)hexyl]-2-(3-chloro-2-ethylsulfonylphenyl)-2-hydroxyethylamine
2-(5-ethyl-2-fluorophenyl)-N-[3-(2-fluorophenyl)propyl]-2-hydroxyethylamine
2-(4-bromo-3-*t*-butylphenyl)-2-hydroxy-N-[1-methyl-3-(4-nitrophenyl)propyl]ethylamine
2-(5-carboxy-2-nitrophenyl)-N-[2-(2-cyanophenyl)ethyl]-2-hydroxyethylamine
2-(4-butylsulfonyl-3-nitrophenyl)-2-hydroxy(3-methylbenzyl)ethylamine
2-(3-amino-2-bromophenyl)-N-[4-(2-ethylphenyl)butyl]-2-hydroxyethylamine
2-hydroxy-N-[3-(4-isopropylphenyl)-1,1-dimethylpropyl]-2-(5-propoxy-2-propylphenyl)ethylamine
N-[3-(4-*s*-butylphenyl)propyl]-2-hydroxy-2-(2-hydroxy-5-methoxyphenyl)ethylamine
N-[2-(2-butylphenyl)ethyl]-2-hydroxy-2-(3-methyl-4-methylaminophenyl)ethylamine
2-(4-aminocarbonyl-2-isopropylsulfonylphenyl)-2-hydroxy-N-[4-(3-methoxyphenyl)butyl]ethylamine
N-[3-(4-ethoxyphenyl)-1,1-dimethylpropyl]-2-hydroxy-2-(2-hydroxy-5-methylpropylaminocarbonylphenyl)ethylamine
2-(3-amino-4-chlorophenyl)-2-hydroxy-N-[2-(2-propoxyphenyl)propyl]ethylamine
2-(2-amino-5-carboxyphenyl)-2-hydroxy-N-(4-isobutoxybenzyl)ethylamine
N-[5-(3-*t*-butoxyphenyl)pentyl]-2-(4-fluoro-3-dimethylaminophenyl)-2-hydroxyethylamine
2-(3-aminocarbonyl-2-methylpropylaminophenyl)-N-[3-(4-formylphenyl)-1-ethylpropyl]-2-hydroxyethylamine
N-[2-(2-acetylphenyl)ethyl]-2-(3-carboxy-4-ethylisobutylaminocarbonylphenyl)-2-hydroxyethylamine
2-(3-*s*-butylsulfonyl-5-propylaminocarbonylphenyl)-2-hydroxy-N-[3-(4-propionylphenyl)propyl]ethylamine
N-[6-(3-butyrylphenyl)hexyl]-2-hydroxy-2-(3-diisopropylamino-2-methylaminocarbonylphenyl)-2-hydroxyethylamine
2-hydroxy-2-(5-isopropoxy-2-nitrophenyl)-N-[3-(4-[2-methylpropionyl]phenyl)-2-methylpropyl]ethylamine
2-hydroxy-N-(4-methoxycarbonylbenzyl)-2-phenylethylamine
N-[3-(4-ethoxycarbonylphenyl)-1,1-dimethylpropyl]-2-hydroxy-2-(4-isopropylsulfonylphenyl)ethylamine
2-hydroxy-2-phenyl-N-[2-(2-propoxycarbonylphenyl)ethyl]ethylamine
N-[4-(4-butoxycarbonylphenyl)butyl]-2-hydroxy-2-(3-dimethylaminophenyl)ethylamine
2-hydroxy-N-(4-hydroxybenzyl)-2-(4-hydroxyphenyl)ethylamine
N-[3-(3-carboxyphenyl)propyl]-2-hydroxy-2-phenylethylamine
N-[4-(3-aminophenyl)butyl]-2-(4-ethylsulfonylphenyl)-2-hydroxyethylamine
N-[2-(4-aminocarbonylphenyl)propyl]-2-hydroxy-2-(3-isopropylphenyl)ethylamine
2-hydroxy-N-(4-methylaminobenzyl)-2-(4-dimethylaminocarbonylphenyl)ethylamine
N-[3-(4-diethylaminophenyl)pentyl]-2-hydroxy-2-phenylethylamine
2-hydroxy-N-[2-(2-isopropylaminophenyl)ethyl]-2-(4-propylaminophenyl)ethylamine
N-[3-(4-bis-[*s*-butyl]aminophenyl)-1,1-dimethylpropyl]-2-hydroxy-2-(4-nitrophenyl)ethylamine
N-(2-butylaminobenzyl)-2-hydroxy-2-(3-propylaminophenyl)ethylamine
2-hydroxy-2-(3-isopropylphenyl)-N-[2-(4-dimethylaminocarbonylphenyl)ethyl]ethylamine
2-hydroxy-N-[3-(4-methylaminocarbonylphenyl)propyl]-2-phenylethylamine
2-(4-carboxyphenyl)-2-hydroxy-N-[4-(2-propylaminophenyl)butyl]ethylamine
2-hydroxy-N-[3-(diisobutylaminocarbonylphenyl)propyl]-2-(4-propylphenyl)ethylamine
N-[5-(4-*t*-butylaminocarbonylphenyl)pentyl]-2-hydroxy-2-phenylethylamine
N-[3-(2,4-dichlorophenyl)propyl]-2-(2,4-dichlorophenyl)-2-hydroxyethylamine
N-[2-(3,5-difluorophenyl)ethyl]-2-(2-fluorophenyl)-2-hydroxyethylamine
2-hydroxy-N-[3-(2,6-dinitrophenyl)propyl]-2-phenylethylamine
N-[4-(2,5-dicyanophenyl)pentyl]-2-hydroxy-2-(3-nitrophenyl)ethylamine
2-hydroxy-N-(2,6-dimethylbenzyl)-2-phenylethylamine
N-[3-(2,4-dibutylphenyl)-1-methylpropyl]-2-(4-ethylphenyl)-2-hydroxyethylamine
2-hydroxy-N-[3-(2,4-dimethoxyphenyl)-1-methylpropyl]-2-(4-propylaminophenyl)ethylamine
2-hydroxy-N-[2-(3,5-diisobutoxyphenyl)ethyl]-2-(4-methylsulfonylphenyl)ethylamine
2-(3-ethoxyphenyl)-N-[3-(3,5-diformylphenyl)butyl]-2-hydroxyethylamine
N-[4-(2,5-dibutyrylphenyl)pentyl]-2-hydroxy-2-phenylethylamine
2-hydroxy-2-(4-methylaminophenyl)-N-[3-(2,4-dimethoxycarbonylphenyl)-1-methylpropyl]ethylamine
N-[2-(3,5-bis[*s*-butoxycarbonyl]phenyl)-1-methylethyl]-2-(2-ethoxyphenyl)-2-hydroxyethylamine

2-hydroxy-N-[3-(2,6-dihydroxyphenyl)-1,2-dimethylpropyl)-2-(4-hydroxyphenyl)ethylamine
N-[2-(2,5-dicarboxyphenyl)propyl]-2-hydroxy-2-phenylethylamine
N-(3,4-diaminobenzyl)-2-hydroxy-2-(2-fluorophenyl)ethylamine
N-[2-(3,4-diaminocarbonylphenyl)ethyl]-2-(4-butylsulfonylphenyl)-2-hydroxyethylamine
2-hydroxy-N-[3-(3,5-bis[dimethylamino]phenyl)-1-methylpropyl]-2-hydroxy-2-phenylethylamine
2-hydroxy-N-[4-(3,5-bis[methylpropylamino]phenyl)butyl]-2-(4-propylaminophenyl)ethylamine
N-[6-(2,5-bis[diethylaminocarbonyl]phenyl)hexyl]-2-hydroxy-2-(3-hydroxyphenyl)ethylamine
2-hydroxy-2-(2-isopropylphenyl)-N-[4-(3,4-bis[isopropylaminocarbonyl]phenyl)-1-methylbutyl]ethylamine
N-[2-(2-bromo-5-chlorophenyl)ethyl]-2-hydroxy-2-phenylethylamine
N-[3-(5-bromo-2-nitrophenyl)propyl]-2-(4-ethylaminocarbonylphenyl)-2-hydroxyethylamine
N-[4-(3-cyano-2-fluorophenyl)-1-methylbutyl]-2-hydroxy-2-(3-propylaminophenyl)ethylamine
N-[2-(4-cyano-3-nitrophenyl)propyl]-2-hydroxy-2-phenylethylamine
N-[2-(3-bromo-2-propylphenyl)propyl]-2-hydroxy-2-(4-nitrophenyl)ethylamine
N-[3-(2-chloro-4-methylphenyl)-2-methylpropyl]-2-hydroxy-2-(3-propylaminophenyl)ethylamine
N-(4-*t*-butyl-3-nitrobenzyl)-2-hydroxy-2-phenylethylamine
2-hydroxy-N-[4-(3-methoxy-4-propylphenyl)butyl]-2-(4-propylaminocarbonylphenyl)ethylamine
N-[3-(4-bromo-2-*s*-butoxyphenyl)propyl]-2-(3-ethylsulfonylphenyl)-2-hydroxyethylamine
N-[3-(2-amino-4-ethoxyphenyl)propyl]-2-hydroxy-2-(3-nitrophenyl)ethylamine
N-[4-(4-acetyl-2-ethoxycarbonylphenyl)butyl]-2-(3-ethoxyphenyl)-2-hydroxyethylamine
N-[5-(3-butyryl-2-hydroxyphenyl)pentyl]-2-(4-carboxyphenyl)-2-hydroxyethylamine
N-[2-(4-carboxy-3-hydroxyphenyl)ethyl]-2-hydroxy-2-(4-isopropylmethylaminocarbonylphenyl)ethylamine
N-[3-(4-aminocarbonyl-2-carboxyphenyl)propyl]-2-hydroxy-2-(4-propylaminophenyl)ethylamine
N-[4-(3-butoxy-5-dimethylaminophenyl)-1-methylbutyl]-2-hydroxy-2-(2-fluorophenyl)ethylamine
N-[2-(2-acetyl-4-propylaminocarbonylphenyl)-1-methylpropyl]-2-hydroxy-2-(4-methylphenyl)ethylamine
N-[3-(4-butoxycarbonyl-2-cyanophenyl)-1-methylbutyl]-2-hydroxy-2-(3-methoxyphenyl)ethylamine
2-(3-ethylaminophenyl)-N-[3-(3-diethylaminocarbonyl-4-fluorophenyl)-1-methylpropyl]-2-hydroxyethylamine
N-[2-(3-butylmethylaminocarbonyl-4-nitrophenyl)-1-methylpropyl]-2-hydroxy-2-(3-isobutylaminocarbonylphenyl)ethylamine
N-(3-bromo-2-butylaminobenzyl)-2-(3,5-diethoxyphenyl)-2-hydroxyethylamine
N-[3-(4-cyano-3-propionylphenyl)-1,1-dimethylpropyl]-2-hydroxy-2-phenylethylamine
N-[2-(2-cyano-4-ethylisopropylaminocarbonylphenyl)ethyl]-2-hydroxy-2-(3-propylaminophenyl)ethylamine
N-[3-(3-aminocarbonyl-5-nitrophenyl)-1-methylpropyl]-2-hydroxy-2-(2-fluorophenyl)ethylamine

It will be understood that the silylated intermediates provided by this invention are also exemplified by the above group of amine products, since each process proceeds through a silylated intermediate. The following silyl groups are typical of those borne by silylated intermediates.

triethylsilyl
tributylsilyl
dibutylphenylsilyl
methyldipropylsilyl
diphenylpropylsilyl
diethylphenylsilyl
*t*-butyldimethylsilyl
isopropyldiethylsilyl
methyldiphenylsilyl
butylmethylphenylsilyl

Certain classes of the amines and silylated intermediates prepared by this invention constitute preferred classes. Such classes gain their preferred status because of the amines' activity as pharmaceuticals, and not because of any properties of the present process itself, which is equally useful for preparing all of the products. One preferred class includes the compounds wherein n is 0 or 1, and particularly wherein n is 0, or wherein n is 1 and R is chloro, bromo, fluoro or hydroxy. Another preferred class includes the compounds wherein $R^1$ is an alkyl group substituted with phenyl or substituted phenyl. A particularly desirable class includes such compounds wherein the alkyl group is of 3—5 carbon atoms, and especially wherein it is a propyl group substituted with 1 or 2 branching methyl groups. The phenyl ring of such $R^1$ groups is preferably substituted with hydroxy, aminocarbonyl, alkanoyl or carboxy groups, especially hydroxy or aminocarbonyl groups.

The products of the process of this invention are pharmaceuticals, and are useful, as is known in the art, as stimulants and blockers of the β-adronergic receptor system, and as cardiovascular agents. See, for example, U.S. Patents 3,816,516, of Cox, 4,000,193, of Lunts et al., 4,066,755, of Lunts, 4,101,759, of Hartley et al., and European Patent Application 0006735, of Ainsworth et al.

European Patent Application No. 79301278.2 discloses a group of optically active products of the formula

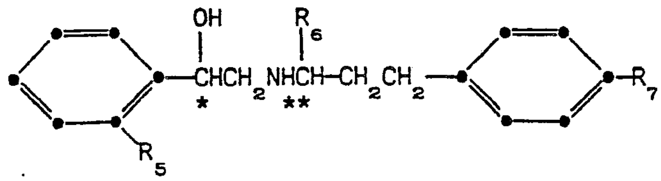

wherein:

$R_5$ is hydrogen or fluorine;

$R_6$ is hydrogen, methyl or ethyl;

$R_7$ is hydroxy, $C_1$—$C_4$ alkanoyloxy, aminocarbonyl, methylaminocarbonyl, or $C_1$—$C_2$ alkoxycarbonyl; with the limitation that when $R_6$ is hydrogen, $R_7$ is aminocarbonyl, methylaminocarbonyl or $C_1$—$C_2$ alkoxycarbonyl;

$C_{*}$ is an asymmetric carbon atom having the R absolute stereochemical configuration;

$C_{**}$ is an asymmetric carbon atom when $R_6$ is methyl or ethyl, and when asymmetric is of the S absolute stereochemical configuration; and

the non-toxic pharmaceutically acceptable acid addition salts thereof.

The above optically active compounds are disclosed to be useful as anti-obesity agents.

European Patent Application No. 79301280.8 discloses another group of ethylamines, described as inotropic agents used for the treatment of cardiac conditions, of the following formula.

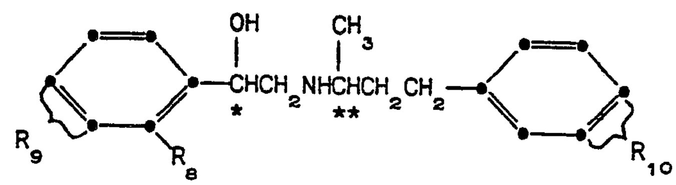

wherein:

$R_8$ is hydrogen or fluorine;

$R_9$ is hydrogen or hydroxy;

$R_{10}$ is hydrogen, hydroxy, fluorine, aminocarbonyl, methylaminocarbonyl, methoxycarbonyl or acetoxy;

$C_{*}$ and $C_{**}$ both are asymmetric carbon atoms having the R absolute stereochemical configuration;

with the limitation that at least one of $R_8$ and $R_9$ is hydrogen; and

the non-toxic pharmaceutically acceptable acid addition salts thereof.

European Patent Application No. 79301281.6 discloses another group of cardiotonic drugs of the inotropic type, having the formula

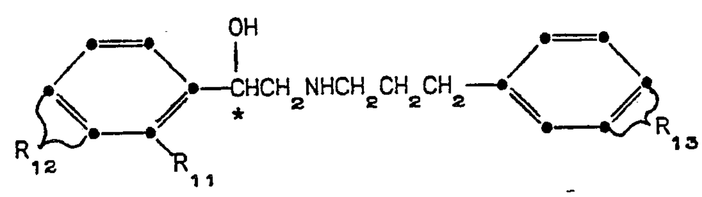

wherein:

$R_{11}$ is hydrogen or fluorine;

$R_{12}$ is hydrogen or hydroxy; provided that at least one of $R_{11}$ and $R_{12}$ is hydrogen;

$R_{13}$ is hydroxy;

$C_{*}$ is an asymmetric carbon atom having the R absolute stereochemical configuration; and the non-toxic pharmaceutically acceptable acid addition salts thereof.

Finally, European Patent Application No. 79301282.4 discloses a class of anti-tumor compounds having the formula

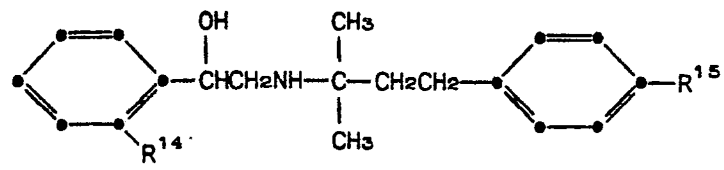

wherein:

$R^{14}$ is hydrogen or fluorine;

$R^{15}$ is hydroxy, aminocarbonyl, methylaminocarbonyl, dimethylaminocarbonyl, acetylamino or methanesulfonylamino;

and the non-toxic pharmaceutically acceptable acid addition salts thereof.

The starting compounds used in the present invention are readily prepared by conventional procedures. As noted above, any free silylatable groups on the starting compounds are to be silylated before the process begins. The silyl groups and the silylating process are as described below in the discussion of silylating the amine starting compound.

The process of this invention is used to prepare 2-hydroxy-2-phenylethylamines, and its advantage lies in its ability to give a high degree of ring opening of the epoxide starting compound, to give a high degree of attack at the β-carbon of the opened epoxide, and to transfer the silyl group to the oxygen atom of the opened epoxide, preventing further reaction at that site. The result of these benefits is seen in the fact that the process of this invention produces excellent yields of the desired products.

The present process has no effect on the stereochemistry of its products. When the products have asymmetric centers, the stereochemistry of them is the same as the stereochemistry of the corresponding centers of the starting materials. In this document, if the stereochemistry of specific compounds is not indicated, it will be understood that the natural mixture of stereoisomers is meant.

It is not necessary to use a significant excess of either starting material of the present process. As the examples below indicate, the process has usually been operated with a small excess, in the range of about 5—20%, of the styrene oxide, to assure that the more expensive and difficult to obtain silylated amine was fully used. However, the use of an excess of either starting material is not necessary and the process may be effectively operated at stoichiometric proportions. The use of an excess, even a very large excess, of either starting compound is not harmful, however, and such may be used as desired.

It will be understood, however, that the preparation of the silylated amine starting compound can be seriously upset by the presence of small amounts of water in the reaction mixture, since silylating agents will consume substantially all available water before they react with any other compound. It is therefore advisable to use very dry starting materials and solvent, to measure the amount of water present, and to add an appropriate excess of silylating agent to take up the water present in the mixture. The reader will understand that very careful drying of the equipment and inert gas blanketing of the reactor are clearly advisable.

The amine starting compound used in this invention is silylated with a tri-substituted silyl group, such as have often been used before in synthetic organic chemistry. The silyl group bears at least one $C_1$—$C_4$ alkyl group, and the other two substituents on it may be either phenyl or $C_1$—$C_4$ alkyl. The most widely used such silyl group is undoubtedly trimethylsilyl; other groups are equally applicable, of course, such as diphenyl-*t*-butyl, triethyl, tributyl, propyldiphenyl and the like. The silylated amine is prepared, as will be understood by the ordinarily skilled reader, by simple reaction of the appropriate amine with a suitable silylating agent containing the tri-substituted silyl group. Such reagents include, for example, N-(tri-substituted)silylimidazole, N-(trisubstituted)silylacetamide, bis(tri-substituted silyl)acetamide, and N,O-bis(tri-substituted silyl)acetamide. Such silylating agents are common commercial products, and the choice of silylating agent used to prepare the silylated amine is not a critical matter. Any such silylating agent will serve.

The reaction of this invention is carried out either neat — without a solvent — or in dimethylsulfoxide, preferably in dimethylsulfoxide. The concentration of the reactants in dimethylsulfoxide, when it is used, is not critical; any amount of the solvent great enough to dissolve the reactants is adequate, and there is no need to operate the process more dilute. It has been found convenient to use an amount of dimethylsulfoxide in the range of 300—1000 ml. per g-mole, but other amounts of solvent may be used as the operator may find convenient in a given instance.

It is preferred to carry out the process at a temperature in the range of about 55—85°. It appears that yields of the desired product are somewhat better when the process is operated at relatively low temperatures; the throughput of the process, however, is greater when it is operated at relatively high temperatures to increase the reaction's speed. Thus, the operator must take into account the relative values of high yield and high throughput to his particular circumstances in choosing the optimum temperaure, as is usually necessary in organic chemical processes. The process may be operated at temperatures both higher and lower than the preferred range just stated, such as the range from about the ambient temperature to about 100°, but it will be found that temperatures below about 55° provide undesirably slow reactions, and that temperatures above about 85° give an amount of by-products which will usually be undesirable.

Reaction of the epoxide and the silylated amine provides a silylated intermediate, which is the desired product bearing the silyl group of the silylated amine on its hydroxy oxygen. This intermediate is easily hydrolyzed to give the desired product. It is very well known that silyl protecting groups are easily hydrolyzed to remove them from the protected group; the usual procedures are used in this process. It is preferred to hydrolyze the silyl group in dilute aqueous or alkanolic acid, most preferably a hydrohalic acid. However, it is entirely possible to hydrolyze the more labile silyl groups in water alone, or in an alkanol alone or in dilute aqueous base. The usual alkanol solvents such as methanol, ethanol, propanol and the like are suitable. For example, reagents such as aqueous hydrochloric acid, hydrobromic acid, sulfuric acid, nitric acid, phosphoric acid, sodium hydroxide, potassium hydroxide, potassium carbonate, potassium bicarbonate, acetic acid and the like are used. Fluoride ion is the most general silyl group cleaving agent and is used even for the few groups, such as diphenyl-*t*-butylsilyl, which are resistant to acid hydrolysis. It is preferable to carry out the hydrolysis at a moderate temperature in the range of from about 0° to about 50°, preferably from about the ambient temperature to about 50°. When silylated hydroxy, amino and the like groups are on the starting compounds, those silyl groups are also cleanly hydrolyzed in the same step.

When the hydrolysis step of the process is carried out under acid conditions, the product will be obtained as a salt of the acid, which may be converted back to the free base, in the usual manner, by treatment under mild basic conditions. The formation of such acid addition salts of pharmaceutical amines is very common and well understood, and so is the conversion to the free base of the compounds. The salts of these compounds are in no way unusual.

The periods of time necessary to carry out the process of this invention are highly variable, depending on the temperatures at which the operator chooses to run. When a temperature in the preferred range of about 55—85° is used, typical reaction times are in the range of about 10—48 hours. It will be understood that, of course, the optimum time at a given temperature depends on the relative importance of throughput and yield to the operator. The hydrolysis step is quite rapid, and usually reaction times in the range of from a few minutes to a few hours are quite adequate. The examples below provide further information about the reaction times and the results provided thereby.

It has been found that it is possible to prepare the silylated amine and to react it with the styrene oxide by simply adding the amine, the silylating agent and the styrene oxide to a reactor all at once. In general, it is advisable to prepare the silylated amine first, and then to add the styrene oxide to it. It is unnecessary to isolate and purify the silylated amine before adding styrene oxide, and it is also unnecessary to isolate the silylated intermediate before it is hydrolyzed. As the examples below indicate, the process goes very well without intermediate isolation, in "one pot" style.

It is relatively easy to distinguish the desired product derived from β-attack of the styrene oxide from the undesired α-isomer. The best distinguishing method is nuclear magnetic resonance (nmr) analysis in which the 2-methynyl proton of the desired product is seen at about δ4.7 in deuterated chloroform with trimethylsilane as the internal standard, as a well-resolved doublet of doublets. The same proton in the undesired product is seen as a multiplet at about δ3.4—3.8, overlapping the signals of the two adjacent protons. Thin layer chromatography on silica has also been characteristic; in all cases observed the β-isomer is the more polar, in chloroform/methanol/$NH_4OH$ systems.

The following examples further explain the process of this invention.

Example 1

N-[3-(4-aminocarbonylphenyl)-(S)-1-methylpropyl)]-(S)-2-hydroxy-2-phenylethylamine

A gas-tight 3-necked flask was fitted with a mechanical stirrer, thermometer and nitrogen inlet, and was kept under dry nitrogen during all operations of the following process. To it was added 130.6 g. of distilled N-trimethylsilylacetamide, 225.5 g. of dry dimethylsulfoxide (DMSO), 157.9 g. of dry 3-amino-1-(4-aminocarbonylphenyl)butane (S isomer) and 118.6 g. of dry (−)-styrene oxide. The mixture was warmed with stirring to 73—75°, over a period of about 2 hours. The mixture was stirred at 73—75° for 15 hours, to prepare the silylated intermediate, N-[(S)-3-(4-aminocarbonylphenyl)-1-methylpropyl-(S)-2-trimethylsilyloxy-2-phenylethylamine, and was then cooled slowly to about 30° and poured into 600 ml. of 1.7N hydrochloric acid, at such a rate as to maintain the acid mixture at 45—50°. The mixture was allowed to stand 20 minutes after the addition, and it was then partitioned with 520 ml. of ethyl acetate. The aqueous layer was separated, and added over a period of about 1 hour to a stirred solution of 104 g. of sodium hydroxide in 2500 ml. of water at about 0°, after which the mixture was cooled to −8° and stirred slowly for another hour. The crude product was then separated by vacuum filtration, washed with three 50 ml. portions of ice water, and pressed as dry as possible. The wet cake was then dissolved in 1000 ml. of acetonitrile at 65—70°, 150 ml. of 50% aqueous methanol was added, and the mixture was cooled undisturbed for 3 hours at ambient temperature and then overnight at 0°. The precipitate was filtered, washed with 50 ml. of 0° acetonitrile, and redissolved in 1000 ml. of boiling acetonitrile containing 180 ml. of 80% aqueous methanol. The mixture was allowed to cool slowly with seeding, and to stand for several hours at ambient temperature and then at 0° overnight. Filtration and vacuum drying of the crystalline product provided 142.1 g. of substantially pure product, m.p. 154.5—157°, a yield of 62% of the theoretical yield. The product was the R,S isomer, and was further identified by optical rotation: $[c]_D = -29.8°$, $[\alpha]_{365} = -99.8°$ (1% methanol); UV(MeOH), λ(E)g244 (13,400). Anal. Calcd. for $C_{19}H_{24}N_2O_2$: C, 73.05; H, 7.74;

N, 8.97; O, 10.24; Found: C, 73.29; H, 7.62; N, 8.70, O, 10.41; TLC: $R_f$ = 0.26, one spot on F—254 silica with $CHCl_3$-methanol-concentrated ammonium hydroxide (100:10:1).

Example 2

(S)-2-hydroxy-N-(S)-1-phenylethyl-2-phenylethylamine

To a small round-bottom flask, under nitrogen, were added 7.90 g. of (−)-α-methylbenzylamine, 9.50 g. of N-trimethylsilylacetamide, 10 ml. of DMSO and 7.85 g. of (−)-styrene oxide. The mixture was placed in an oil bath at 65—68° for 44 hours, without stirring, to prepare (S)-2-trimethylsilyloxy-N-(S)-1-phenylethyl-2-phenylethylamine, after which the mixture was poured onto 100 g. of ice containing 10 ml. of concentrated hydrochloric acid. The aqueous phase was washed with 50 ml. of hexane and then added slowly with good stirring to 100 g. of ice-water containing 10 g. of sodium hydroxide at −5°. The white precipitate was filtered and dried at 55° under high vacuum affording a 12.0 g. (80%) yield of the β-isomer, m.p. 82—86°. $[\alpha]_D$ = −83.3, $[\alpha]_{365}$ = −122.7 (1% methanol); TLC (F—254 silica) $R_f$ = 0.30, one spot (9:1, hexane-THF); 0.72 one spot (THF). MS, m/e (rel. intensity): 241 (100), 134 (64), 107 (18); UV (MeOH); λ(E), 258(600). IR(KBr) 3250 (bd), 3083, 3024, 2849, 1087, 1060 ($cm^{-1}$). Anal. Calcd. for $C_{16}H_{19}N_1O_1$: C, 79.63; H, 7.94; N, 5.80; O, 6.63; Found: C, 76.62; H, 8.00; N, 5.65; O, 6.63. $^{13}C$ NMR ($CDCl_3$) δ, 145.0, 142.6, 128.5, 128.3, 127.7, 127.4, 127.2, 127.1, 126.6, 126.5, 125.8, 72.0, 57.6, 54.9, 54.8, 24.21. $^1H$ NMR (270 mHz, $CDCl_3$): δ, 7.27 (10H, m), 4.69 (1H, dxd) 3.82 (1H, q), 3.82 (1H, q), 2.79 (1H, dxd), 2.55 (1H, dxd), 135 (3H, d).

Example 3

(S)-2-hydroxy-N-(S)-1-phenylethyl-2-phenylethylamine

The process of Example 2 was followed, starting with 3.3 g. of (−)-α-methylbenzylamine, 4.10 g. of N-trimethylsilylacetamide and 3.6 g. of (−)-styrene oxide at 65° for 24 hours. No solvent was used. Treatment of the mixture after the process as described in Example 2 was carried out to afford 5.14 g. (79%) of material which, by comparative thin layer chromatography and nuclear magnetic resonance analysis, was indistinguishable from the product described in Example 2.

Example 4

N-benzyl-(S)-2-hydroxy-2-phenylethylamine

To a dry 50 ml. round-bottom flask was added 16 ml. of DMSO, 9.94 g. of N-trimethylsilylacetamide, 6.80 g. of benzylamine and 8.4 g. of (−)-styrene oxide. The flask was sealed with a membrane, put under nitrogen pressure and heated at 60—65° for 20 hours to prepare N-benzyl-(S)-2-trimethylsilyloxy-2-phenylethylamine. The reaction was then quenched by adding 1% hydrochloric acid in methanol until the pH was 2. That mixture was stirred for 5 minutes, and its pH was adjusted to about 12 by addition of 6N sodium hydroxide. The mixture was then extracted twice with 100 ml. portions of ethyl acetate, and the organic layers were washed twice with 50 ml. portions of water and saturated sodium chloride solution. The organic layer was then dried over magnesium sulfate, and evaporated to dryness under vacuum to obtain 14.6 g. of crude product. A 500 mg. aliquot of the crude product was diluted with 20 ml. of dry methanol, and was analyzed by high performance reverse phase chromatography, on a Waters instrument, using a 10 cm. column packed with C—18 bondapak, and eluting with a solvent composed of 70/30/1 water/methanol/dibutylammonium phosphate buffer. The analysis showed that the ratio of the desired β-substituted product to the undesired α-substituted product was about 3.8:1. The analytical yield of the β isomer was about 64% vs. a pure, analytical standard.

Example 5

(S)-2-hydroxy-N-[2-(diphenyl-*t*-butylsilyloxy)ethyl]-2-phenylethylamine

To a 50 ml. round-bottom flask, under nitrogen, were added 5 ml. of DMSO, 2.60 g. of N-trimethylsilylacetamide, 5.64 g. of diphenyl-*t*-butylsilyl 2-aminoethyl ether, and 2.20 ml. of (−)-styrene oxide. The mixture was heated under nitrogen at 65° for 25 hours to prepare (S)-2-trimethylsilyloxy-N-[2-(diphenyl-*t*-butylsilyloxy)ethyl]-2-phenylethylamine, and was then diluted with methanol and its pH was adjusted to 4—5 with acetic acid. The volatile portions of the mixture were then removed under vacuum, and the residue was diluted with ethyl acetate. The solution was washed with 5% aqueous sodium bicarbonate and dried over sodium sulfate. It was then evaporated to dryness under vacuum to obtain 7.56 g. of crude product. A 560 mg. aliquot of the crude product was purified by preparative thick layer chromatography on silica gel, eluting with THF-hexane-methanol (100:10:1), to obtain 0.40 g. of purified product, indicating a yield of 69% of the theoretical yield. The product was identified by IR (neat), 3600, 3400 (bd), 1430, 1113, 1094, 1064, 740, 701 $cm^{-1}$; $^1H$ NMR ($CDCl_3$) δ = 7.6 (m, 4H), 7.25 (m, 13H), 4.62 (dxd, 1H), 3.4—3.8 (m, 3H), 3.08 (bd s, 2H, $D_2O$ exchanges), 2.78 (m, 2H), 1.08 (s, 9H), 0.91 (d, 3H); TLC, $R_f$ = 0.5 (hexane-THF-methanol, 100—10—1), one spot; $[\alpha]_D$ = −5.8°, $[\alpha]_{365}$ = −20° (1% in methanol).

The protecting group may be removed by reaction with fluoride ion, as taught by Hanessian and Lavallee, *Can. J. Chem. 53*, 2975 (1975) and *55*, 562 (1977), to obtain (S)-2-hydroxy-N-(2-hydroxyethyl)-2-phenylethylamine.

Example 6

N-benzyl-2-hydroxy-2-phenylethylamine

To a round-bottom flask under nitrogen were added 7.4 g. of N-trimethylsilylacetamide, 5.60 ml. of benzylamine, and 6.72 g. of d,l-styrene oxide. The mixture was heated at 55—57° for 24 hours to form the same silylated intermediate as Example 4, after which 10 ml. of concentrated hydrochloric acid was added, with cooling, and methanol was added until the mixture became a solution. After 10 minutes of standing at 25°, the mixture was slowly poured into 200 g. of ice and 200 ml. of 1N sodium hydroxide at about 5°, and the mixture was filtered. The solids were washed with two 50 ml. portions of ice water, and vacuum dried at 55° to obtain 7.74 g. of crude product, which was analyzed by thin layer chromatography, $R_f = 0.19$, main spot (β isomer); $R_f = 0.23$, trace (α isomer) (silica gel with methylene chloride-methanol-concentrated ammonium hydroxide, 250:25:2); $^1H$ NMR (4:1 DMSO-$d_6$—$CDCl_3$): δ7.50 (unresolved, 10H), 4.71 (1H, dxd, Jα-β = 5, Jα-β′ = 5), 3.78 (2H, s), 2.80 (2H, dxd, Jβ-β = 7, Jβ-α = 5), 1.60 (2H, broad, $D_2O$ exchanges).

Example 7

N-benzyl-2-hydroxy-2-phenylethylamine, and hydrochloride

The process of this example was carried out exactly as was Example 6, except for the amounts of the reactants, which were 16 ml. of DMSO, 6.18 g. of benzylamine, 8.39 g. of N-trimethylsilylacetamide, and 7.6 g. of d,l-styrene oxide.

The product was isolated as described in Example 6, affording 8.63 g. of pure free base. Analysis of it by thin layer chromatography and nuclear magnetic resonance analysis showed that the material was pure. TLC; one spot, R = 0.19 (silica with methylene chloride-methanol-concentrated ammonium hydroxide, 250:25:2.)

For further characterization, 2.27 g. of this free base was treated with 1 equivalent of hydrochloric acid in methanol affording 2.38 g. of the corresponding hydrochloride salt, m.p. 227—231° (after recrystallization from acetone); UV (in ethanol), λ(E) 205 (14,400), 250 (550), 255 (650), 262 (550), 268 (200). IR (KBr), 3400 (broad), 2800 (broad), 1458, 1072 $cm^{-1}$. Anal. Calcd. for $C_{15}H_{18}N_1O_1$: C, 68.30; H, 6.88; N, 5.31; O, 6.07; Cl, 13.44; Found: C, 68.10; H, 6.87; N, 5.10; O, 6.10; Cl, 13.39.

Example 8

N-cyclohexyl-(S)-2-hydroxy-2-phenylethylamine, hydrochloride

To a round-bottom flask under nitrogen were added 35.5 ml. of DMSO, 24.7 g. of N-trimethylsilylacetamide, 16.3 g. of cyclohexylamine, and 20.7 g. of (−)-styrene oxide. The mixture was stirred magnetically under nitrogen for 43 hours at 55°, to form N-cyclohexyl-(S)-2-trimethylsilyloxy-2-phenylethylamine, and the mixture was then diluted with 40 ml. of methanol and cooled to about 5°. To it was then added 16.6 ml. of concentrated hydrochloric acid, and 50 ml. of hexane. After 20 minutes of stirring, the mixture was filtered and the solids were dried to obtain 13.75 g. of product, m.p. 179—182°, which was found to be one spot by thin layer chromatography (F—254 silica; chloroform-methanol-concentrated ammonium hydroxide, 100:10:1); $[\alpha]_D = -50.5°$; $[\alpha]_{365} = -159.3°$ (1% methanol). IR (< Br) 3278 (broad), 3000—2400 (broad). UV (methanol), 206 (8,100), 214 (sh, 4,400), 255 (300). Anal. Calcd. for $C_{14}H_{22}ClNO$: C, 65.74; H, 8.67; N, 5.48; O, 6.25; S, 0.00; Cl, 13.86; Found: C, 66.05; H, 8.64; N, 5.76; O, 6.23; S, none; Cl, 13.80.

An additional 20.01 g. of the corresponding free base was obtained in about 80% purity by the addition of the above acidic aqueous layer to 200 ml. of 1N NaOH, filtration, and vacuum drying. Trituration of 1.20 g. of this product with 2.00 ml. of dry acetone gave 0.85 g. of pure material, m.p. 108—111°. TLC: $R_f = 0.67$, one spot (chloroform-methanol-ammonium hydroxide, 250:50:7.5); $[\alpha]_D = -53.4°$, $[\alpha]_{365} = -170.5°$ (1% in chloroform); MS: m/e (rel. intensity) 189 (20), 113 (100). Anal. Calcd. for $C_{14}H_{21}NO$: C, 76.67; H, 9.65; N, 6.39; O, 7.29; Found: C, 76.44; H, 9.48; N, 6.34; O, 7.42. $^{13}C$ NMR ($CDCl_3$) δ, 143.1, 128.3, 127.1, 125.8, 72.1, 56.5, 54.3, 33.8, 33.6, 26.1, 25.0.

Example 9

N-cyclohexyl-(S)-2-hydroxy-2-phenylethylamine

To a nitrogen-blanketed flask were added 2.7 ml. of DMSO, 0.67 g. of cyclohexylamine, 1.24 g. of N-trimethylsilylacetamide and 0.89 g. of (−)-styrene oxide. The mixture was heated to 75° with stirring, and was held at that temperature for 41 hours to form the silylated intermediate. The DMSO was then removed under vacuum at about 60°, the residue was cooled, and 10 ml. of methanol and 10 ml. of 1N hydrochloric acid were added. The mixture was stirred for 10 minutes at about 30°, and then 10 ml. of 2N potassium hydroxide was added. The mixture was cooled and filtered, and the solids were washed with water and vacuum dried to obtain 1.10 g. of the desired product, m.p. 107—111°. Analysis by nuclear magnetic resonance and thin layer chromatography showed that this material was identical to material purified by chromatography. $^1H$ NMR (DMSO-$d_6$); δ 7.32 (5H, s), 4.60 (1H, dxd), 2.70 (2H, dxd), 2.6—2.0 (3H, unresolved, 2H exchange with $D_2O$), 1.8—1.0 (11H, unresolved). MS: m/e, 219 ($M^+$) 189, 113 (base).

Example 10

N-benzyl-2-hydroxy-2-(3-methoxyphenyl)ethylamine

A 50 ml. round-bottom flask was charged with 1.09 ml. of benzylamine, 1.44 g. of N-trimethylsilylacetamide, 2.5 ml. of DMSO and 1.65 g. of 3-methoxystyrene oxide. The mixture was heated to 65° for a short

time under nitrogen, and then was held at 50° overnight. It was then held at 65° for about 8 hours, cooled to ambient temperature overnight, reheated to 65° for 4.5 hours, and held at that temperature until the mixture had been at 65° for a total of 17 hours to form N-benzyl-2-trimethylsilyloxy-2-(3-methoxyphenyl)ethylamine. To it was then added 2.7 ml. of methanol, and the mixture was then added to 33 ml. of 1N sodium hydroxide. The aqueous mixture was stirred for a time, and was then extracted with two 100 ml. portions of ethyl acetate. The organic layers were combined, dried over sodium sulfate and evaporated to dryness under vacuum. The residue was analyzed by thin layer chromatography, and was found to be primarily composed of the β-alkylation product. Only small spots were seen for the α-isomer and the bis-alkylation products.

Example 11

N-benzyl-2-(3,4-dichlorophenyl)-2-hydroxyethylamine

A mixture of 1.09 ml. of benzylamine, 1.44 g. of N-trimethylsilylacetamide, 2.5 ml. of DMSO, and 2.08 g. of 3,4-dichlorostyrene oxide was prepared in a nitrogen-blanketed 50 ml. flask, and was heated to 65° for about 8 hours, and then at 50° overnight. It was reheated to 65° and held for 4 hours to form N-benzyl-2-(3,4-dichlorophenyl)-2-trimethylsilyloxyethylamine, and was then added to 7.62 ml. of water and 0.95 ml. of concentrated hydrochloric acid. To it was added 2.7 ml. of methanol, and then 60 ml. of 1:1 hexane-diethyl ether. The mixture was filtered, the layers of the filtrate were separated, and the filtered precipitate was added to the aqueous phase. To the aqueous phase was then added 33 ml. of 1N sodium hydroxide at about 0°, and the mixture was stirred at that temperature for one hour. It was then extracted with 100 ml. of ethyl acetate, and then again with 100 ml. of ethyl acetate, after 2 g. of sodium chloride had been added. The two organic layers were combined, dried with sodium sulfate, and evaporated to dryness under vacuum to obtain 2.72 g. of crude product. It was analyzed by thin layer chromatography and found to be predominantly composed of the β-isomer. Traces of the α-isomer and bis-alkylated product were seen.

Example 12

N-cyclohexyl-2-hydroxy-2-(4-methylphenyl)ethylamine, and hydrochloride

A mixture of 5.18 g. of N-trimethylsilylacetamide, 3.80 ml. of cyclohexylamine, 3.4 ml. of DMSO, and 4.83 g. of 4-methylstyrene oxide was prepared in a nitrogen-blanketed flask, and was stirred at 50° for 48 hours to form N-cyclohexyl-2-trimethylsilyloxy-2-(4-methylphenyl)ethylamine. To the mixture was then added 40 ml. of methanol, the mixture was cooled to about 5° and to it was added dropwise 16.6 ml. of concentrated hydrochloric acid. Fifty ml. of hexane was then added, and the mixture was stirred for 20 minutes and filtered, affording 3.93 g. of the pure hydrochloride salt, m.p. 204—209°. UV (methanol), λ(E): 211 (7800), 206 (7800), 220 (sh, 6500), 260 (400); MS: m/e (rel. intensity); 233 ($M^+$—HCl, 100), 120 (19), 112 (74); $^1H$ NMR (DMSO-$d_6$) δ 7.4 (dxd, 5H), 6.25 (2H, broad, exchanges with $D_2O$), 5.15 (1H, broad, exchanges with $D_2O$), 5.05 (1H, dxd), 3.10 (2H, dxd), 2.33 (3H, s) 2.1 (1H, unresolved) 2.0—1.0 (10H, unresolved); Anal. Calcd. for $C_{15}H_{24}ClNO$: C, 66.77; H, 8.97; N, 5.19; O, 5.94; Cl, 13.17; Found: C, 66.95; H, 8.76; N, 5.00; O, 6.16; Cl, 13.36; IR(KBr) 3270 (broad), 3200—2400 (broad) $cm^{-1}$; HPLC analysis at 25 mg./ml. with a 5 μl. injection on a Waters Associates C—18 column with 80:20:1 water-methanol-dibutylammonium phosphate buffer at 3 ml. min. suggested a purity of ca. 99%.

The layers of the above filtrate were then separated, and the aqueous layer was partially evaporated under vacuum to remove the methanol. The rest of the aqueous layer was added to 200 ml. of 1N sodium hydroxide at about 0°, dropwise, and the precipitated product was separated by filtration and vacuum dried to obtain 3.98 g. of the desired product in crude form. A 283 mg. portion of the crude product was purified by thick layer chromatography on silica gel, eluting with chloroform-methanol-ammoniumhydroxide, 210:25:2.5 to obtain 170 mg. of purified desired product, m.p. 86—89°. Anal. Calcd. for $C_{15}H_{23}NO$: C, 77.21; H, 9.94; N, 6.00; O, 6.86; Found: C, 77.12; H, 10.01; N, 5.94: O, 7.04.

Example 13

N-benzyl-2-(2-chlorophenyl)-2-hydroxyethylamine

A mixture of 1.14 g. of benzylamine, 1.67 g. of N-trimethylsilylacetamide, 2.5 ml. of DMSO and 1.80 g. of 2-chlorostyrene oxide was prepared in a nitrogen-blanketed flask, and was stirred at 55—60° for 51 hours to form N-benzyl-2-(2-chlorophenyl)-2-trimethylsilyloxyethylamine. The mixture was then treated with 5 ml. of methanol and 1.1 ml. of concentrated hydrochloric acid, and was extracted with 5 ml. of 1:4 diethyl ether:hexane. One hundred ml. of 1N sodium hydroxide was added to the aqueous layer, and it was then extracted with three 50 ml. portions of diethyl ether and the organic layers were combined, washed with 50 ml. of water and 50 ml. of saturated sodium chloride solution, and dried over sodium sulfate. The ether extract was then evaporated to dryness under vacuum, furnishing 2.35 g. of crude product which was analyzed by HPLC on a Waters Associates instrument on a C—18 Bondapak column with 50:50:1 methanol-water-dibutylammonium phosphate buffer at 4 ml./min. with a sample injection of 250 μg. The estimated yield of desired product was ca. 80%. The hydrochloride salt was prepared by the addition of conc. hydrochloric acid to pH 2 to a solution of 363 mg. of the above free base in 2 ml. of methanol. The volatiles were removed in vacuo at 25° and the residue was triturated with 2 ml. of dry acetone. Filtration and vacuum drying provided 257 mg. of the salt, m.p. 177—185° (d); Anal. Calcd. for $C_{15}H_{17}Cl_2NO$: C, 60.41; H, 5.75; N, 4.70; O, 5.35; Cl, 23.78; Found: C, 60.37; H, 5.69; N, 4.65; O, 5.56; Cl, 23.58; MS (FD), 262

$M^+$ —Cl or $M^+$ —HCl + 1), 261 ($M^+$ —HCl), 120 (base), 107 (10); IR (KBr): 3500—2500, 3190, 2771 $cm^{-1}$; $^1H$ NMR($CDCl_3$—DMSO-$d_6$, 9:1)); δ 7.3 (m, 12H), 5.42 (dxd, 1H), 4.18 (s, 2H), 2.7—3.3 (m, 2H).

Example 14

N-benzyl-2-hydroxy-2-(4-nitrophenyl)ethylamine

A mixture of 9 ml. of DMSO, 3.97 g. of benzylamine, 5.82 g. of N-trimethylsilylacetamide and 6.72 g. of 4-nitrostyrene oxide was prepared in a nitrogen-blanketed flask and stirred at 60—65° for 22 hours to form N-benzyl-2-trimethylsilyloxy-2-(4-nitrophenyl)ethylamine. To it was then added 5 ml. of methanol, and enough concentrated hydrochloric acid to bring the pH to 2. It was then washed with 10 ml. of 1:1 hexane:ethyl acetate, and diluted with 100 ml. of water and made basic to pH 12 with sodium hydroxide. The basic mixture was then extracted three times with 50 ml. portions of ethyl acetate, and the organic layers were combined, dried over magnesium sulfate and evaporated to dryness under vacuum. The residue was then dissolved in a minimum amount of methanol at 40°, made acidic with concentrated hydrochloric acid, cooled to about 0° and filtered, and the solids were dried under vacuum to obtain 7.89 g. of product, m.p. 240—245°, which was one spot by thin layer chromatography (F254 silica gel developed with chloroform-methanol-concentrated ammonium hydroxide, 250:25:1). Anal. Calcd. for $C_{15}H_{17}N_2O_3Cl$: C, 58.35; H, 5.55; N, 9.07; O, 15.54; Cl, 11.48; Found: C, 58.56; H, 5.73; N, 8.90; O, 15.27; Cl, 11.14; MS (FD); m/e, 272 ($M^+$ —HCl). UV (MeOH) 267 (10,950). IR (KBr) 3318, 1521 $cm^{-1}$.

Example 15

N-[(S)-1-phenylethyl]-2-(4-chlorophenyl)-2-hydroxyethylamine

In a nitrogen-blanketed flask were combined 1.30 ml. of DMSO, 0.72 g. of N-trimethylsilylacetamide, 0.56 g. of (S)-α-methylbenzylamine, and 0.77 g. of 4-chlorostyrene oxide. The mixture was stirred at 65—70° for 26 hours to form N-[(S)-1-phenylethyl]-2-(4-chlorophenyl)-2-trimethylsilyloxyethylamine. Two ml. of methanol was then added, and the mixture was made acid to pH 2 with concentrated hydrochloric acid. The mixture was stirred for about 20 minutes, and was then extracted with 10 ml. of diethyl ether and made basic with 6N sodium hydroxide. It was then extracted three times with 20 ml. portions of ethyl acetate, and the organic layers were combined and washed with two 10 ml. portions of water and dried over magnesium sulfate. The volatile constituents were removed under vacuum to obtain 1.17 g. of crude product. Analysis by thin layer chromatography indicated the mixture to be about a 1:1 mixture of diastereomers (silica gel with chloroform-methanol-ammonium hydroxide, 250:25:2.5), $R_f$ = 0.51 and 0.46. The products were obtained as a 60/40 mixture of diastereomers by conversion to the hydrochloride salt with 1 eq HCl in methanol, removal of volatiles and recrystallization from ethanol, $[\alpha]_D = -74.2°$, $[\alpha]_{365} = -272.5°$ (1% in methanol); MS (FD): 275 ($M^+$ —HCl), 134 (base). Anal. Calcd. for $C_{16}H_{19}NOCl_2$: C, 61.55; H, 6.13; N, 4.49; O, 5.12, Cl, 22.71; Found: C, 61.49; H, 5.98; N, 4.23; O, 5.36; Cl, 22.62.

Example 16

N-[3-(4-aminocarbonylphenyl)-1-methylpropyl]-2-(S)-hydroxy-2-phenylethylamine

In a nitrogen-blanketed flask were combined 3.3 ml. of N,O-bis(trimethylsilyl)acetamide, 6.8 ml. of DMSO, 4.65 g. of 3-(4-aminocarbonylphenyl)-1-methylpropylamine, and 3.20 g. of (−)-styrene oxide, and the mixture was stirred at 75° for 15 hours to form N-[3-(4-aminocarbonylphenyl)-1-methylpropyl]-2-(S)-trimethylsilyloxy-2-phenylethylamine. It was then cooled to ambient temperature, diluted with 2:1 methanol:6N hydrochloric acid until acidic, stirred for 5 minutes and evaporated under vacuum. The crude mixture was diluted with 25 ml. of water and extracted with 10 ml. hexane. The acidic aqueous phase was slowly added dropwise to 50 ml. of 1N sodium hydroxide at 0°, and stirred for 10 minutes. The mixture was then filtered cold, and the solids were washed with a small amount of ice water and vacuum dried to obtain 6.50 g. of crude product, which was analyzed by comparative TLC analysis vs the crude reaction mixture of Example 1 on silica gel F254, eluting with chloroform-methanol-ammonium hydroxide, 250:50:5. The product appeared to be substantially identical to that of Example 1.

Example 17

N-[3-(4-aminocarbonylphenyl)-1-methylpropyl]-2-(S)-hydroxy-2-phenylethylamine

A mixture of 10 ml. of DMSO, 6.04 g. of N-trimethylsilylimidazole, 6.90 g. of 3-(4-aminocarbonylphenyl)-1-methylpropylamine and 5.18 g. of (−)-styrene oxide was prepared in a 50 ml. nitrogen-blanketed flask, and was stirred at 75° for 16 hours to form the silylated intermediate. The mixture was worked up as described in the example above, providing 9.97 g. of crude free base. Thin layer chromatographic analysis indicated the product quality to be essentially identical to that described in the example above.

Example 18

N-[3-(4-aminocarbonylphenyl)-1-methylpropyl]-2-(S)-hydroxy-2-phenylethylamine

In a nitrogen-blanketed flask were combined 6 ml. of DMSO-$d_6$, 0.67 g. of N-trimethylsilylacetamide, and 1.06 g. of 3-(4-aminocarbonylphenyl)-1-methylpropylamine, and the mixture was slowly warmed until solution was obtained. A 3 ml. portion of the solution was added to 284 mg. of freshly distilled (−)-styrene oxide, and the mixture was then heated at 85° for 23 hours to form the silylated intermediate. The solvent was then removed under nitrogen, and the residue was dissolved in 6 ml. of tetrahydrofuran, 2 ml. of water

and 0.5 ml. of 6N hydrochloric acid. One ml. of methanol was added, and the mixture was stirred for 3 hours at 25°, and then evaporated under vacuum. The residue was made basic with 10% potassium carbonate solution, and extracted with 25 ml. of ethyl acetate. The organic layer was washed twice with water, and with saturated sodium chloride solution and dried over magnesium sulfate. Evaporation of the mixture gave a white solid which was identified by thin layer chromatography on silica gel F—254 with heptane-tetrahydrofuran-methanoltriethylamine (20:10:2:1) to be nearly pure product versus an authentic, analytically pure sample.

Example 19

N-[3-(4-aminocarbonylphenyl)-1-methylpropyl]-2-(S)-hydroxy-2-phenylethylamine

A 0.68 g. portion of 3-(4-aminocarbonylphenyl)-1-methylpropylamine, 0.51 g. of N-trimethylsilylacetamide and 2.5 ml. of DMSO were combined in a nitrogen-blanketed flask, and warmed to 70°. The mixture was then cooled to 25°, and 0.45 ml. of (−)-styrene oxide was added. The mixture was stirred, always under slight nitrogen pressure, at 75° for 44 hours to form the silylated intermediate, and most of the DMSO was then removed under vacuum at 73°. The mixture was diluted with 10 ml. of methanol and 3.9 ml. of 1N hydrochloric acid, and stirred for 5 minutes. The reaction mixture was then evaporated under vacuum at 40° and analyzed by thin layer chromatography on silica versus an authentic pure sample. The major product of the reaction was the desired product.

Example 20

N-[3-(4-aminocarbonylphenyl)-1-methylpropyl]-2-(S)-hydroxy-2-phenylethylamine

In a 500 ml. single-neck flask, under nitrogen, were combined 41.3 g. of N-trimethylsilylacetamide, 50.3 g. of 3-(4-aminocarbonylphenyl)-1-methylpropylamine and 75 ml. of DMSO. The mixture was stirred and warmed slowly to 55°, and then cooled to 25°. To it was added 41 g. of (−)-styrene oxide, and the mixture was then warmed to 73—75° and stirred at that temperature for 16 hours to form the silylated intermediate. It was then cooled to 15°, and added to 350 ml. of 1N hydrochloric acid at 25°. The mixture was allowed to stand for 20 minutes, and to it was then added 200 ml. of 1:4 ethyl acetate:hexane. The aqueous layer was then added to 450 ml. of 1N sodium hydroxide and 350 ml. of water at about 0° with good agitation, and the mixture was stirred for 15 minutes after the addition and then filtered. The solids were washed with 350 ml. portions of ice water, and then with 100 ml. of hexane. The solids were dried under vacuum at 60° to obtain 78.4 g. of crude product. Quantitative high performance liquid chromatographic analysis versus a pure analytical standard using a Waters μBondapak $C_{18}$ column with water-acetonitrile-ammonium acetate (1400-600-8, v/v/w) indicated a purity of 81%. Two recrystallizations (1800 ml. of methanol-isopropanol-water, 1:9:6, and then 1000 ml. methanol-water, 1:1) afforded 51.2 g. of pure material, m.p. 154—156°. $[\alpha]_D = -28.8°$, $[\alpha]_{265} = -95.3°$ (methanol). Anal. Calcd. for $C_{19}H_{24}N_2O_2$: C, 73.05; H, 7.74; N, 8.97; O, 10.24; Found: C, 72.88; H, 7.48; N, 8.69; O, 10.52. UV (methanol); λ(E), 234.5 (13,500), 215 (sh).

Example 21

N-[3-(4-aminocarbonylphenyl)-1,1-dimethylpropyl]-2-hydroxy-2-phenylethylamine

A 20.6 ml. portion of DMSO, 5.36 ml. of hexamethyldisilazane and 10.3 g. of 3-(4-aminocarbonyl)-1,1-dimethylpropylamine were combined in a flask and heated to 75—80° for 12 hours. The flask was allowed to cool, protected by a calcium chloride drying tube. The mixture was then reheated to 80°, and to it was added 7.04 ml. of styrene oxide. The mixture was stirred at 75—80° for 24 hours to form N-[3-(4-aminocarbonylphenyl)-1,1-dimethylpropyl]-2-trimethylsilyloxy-2-phenylethylamine. It was then cooled to 25°, and to it were added 40 ml. of cold water and 5.2 ml. of concentrated hydrochloric acid. The mixture was stirred for 1 hour, and was then extracted twice with 20 ml. portions of dichloromethane. To the aqueous layer was added 100 ml. of methyl ethyl ketone, and its pH was adjusted to 14 with 50% sodium hydroxide. The 2-phase mixture was vigorously stirred, and the aqueous layer was discarded. The organic layer was washed twice with 20 ml. portions of water, and was then concentrated under vacuum to a solid. A small amount of methanol was added, and was removed under vacuum. The residue was then dissolved in 25 ml. of methanol at 60—65°, and 75 ml. of 65° water was added. The mixture was stirred at 65° for five minutes, and was then slowly cooled to 25°, and then to about 0°. The precipitate was filtered out, washed with 20 ml. of water and dried under vacuum to obtain 11.5 g. of the desired product, m.p. 142.5—146°, the identity of which was confirmed by TLC against standard, elemental analysis, and mass spectrum.

| | Theory | Found |
|---|---|---|
| C | 73.59 | 73.34 |
| H | 8.03 | 8.19 |
| N | 8.58 | 8.37 |

MS, m/e (rel. intensity): 272 ($M^+$, 100), 219 (70).

## Claims

1. A process for preparing an amine of the formula

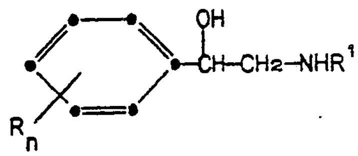

or an acid addition salt thereof; wherein

n is 0, 1 or 2;

the R groups are the same or different, when n is 2, and are chosen from chloro, bromo, fluoro, nitro, $C_1$—$C_4$ alkyl, $C_1$—$C_4$ alkoxy, $C_1$—$C_4$ alkylsulfonyl, hydroxy, carboxy, amino, aminocarbonyl, mono- or di($C_1$—$C_4$ alkyl)amino, or mono- or di($C_1$—$C_4$ alkyl)aminocarbonyl; provided that when n is 2, the R groups are not both *ortho*; provided that an alkoxy R group is not *para*;

$R^1$ is $C_3$—$C_8$ cycloalkyl, or $C_3$—$C_8$ cycloalkyl mono- or disubstituted with $C_1$—$C_4$ alkyl, $C_1$—$C_4$ alkoxy, chloro, bromo or fluoro,

$C_1$—$C_6$ alkyl,

$C_1$—$C_6$ alkyl substituted with chloro, bromo or fluoro,

$C_1$—$C_6$ alkyl monosubstituted with $C_1$—$C_4$ alkoxy, $C_1$—$C_4$ alkanoyl, $C_1$—$C_4$ alkoxycarbonyl, hydroxy, amino, phenyl, or phenyl mono- or disubstituted with chloro, bromo, fluoro, nitro, cyano, $C_1$—$C_4$ alkyl, $C_1$—$C_4$ alkoxy, $C_1$—$C_4$ alkanoyl, $C_1$—$C_4$ alkoxycarbonyl, hydroxy, carboxy, amino, aminocarbonyl, mono- or di($C_1$—$C_4$ alkyl)amino, or mono- or di($C_1$—$C_4$ alkyl)aminocarbonyl;

which process comprises reacting an epoxide of the formula

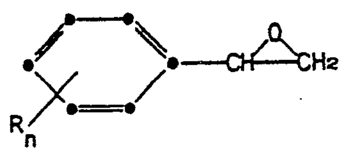

with a silylated amine of the formula

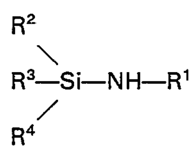

wherein

$R^2$ and $R^3$ are independently phenyl or $C_1$—$C_4$ alkyl, and $R^4$ is $C_1$—$C_4$ alkyl;

provided that carboxy, hydroxy, unsubstituted amino and unsubstituted aminocarbonyl groups on the epoxide and the silylated amine are silylated with the group

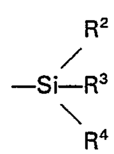 ;

in the presence of dimethylsulfoxide or without a solvent; to prepare a silylated intermediate of the formula

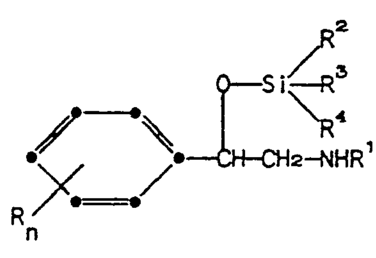

and hydrolyzing the silylated intermediate.

2. A process according to claim 1, wherein n is 0 and $R^1$ is

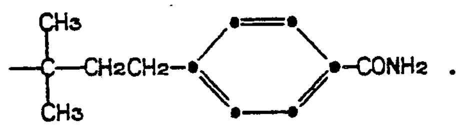

so that N-[3-(4-aminocarbonylphenyl)-1,1-dimethylpropyl]-2-hydroxy-2-phenylethylamine, or an acid addition salt thereof, is prepared.

3. A silylated derivative of the formula

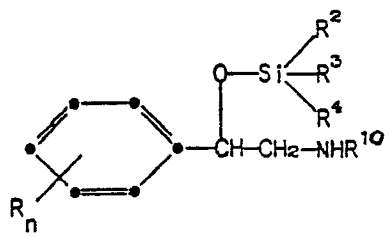

wherein

n is 0, 1 or 2;

the R groups are the same or different, when n is 2, and are chosen from chloro, bromo, fluoro, nitro, $C_1$—$C_4$ alkyl, $C_1$—$C_4$ alkoxy, $C_1$—$C_4$ alkylsulfonyl, hydroxy, carboxy, amino, aminocarbonyl, mono- or di($C_1$—$C_4$ alkyl)amino, or mono- or di($C_1$—$C_4$ alkyl)aminocarbonyl; provided that when n is 2, the R groups are not both *ortho*; provided that an alkoxy R group is not *para*;

$R^{10}$ is $C_3$—$C_8$ cycloalkyl, or $C_3$—$C_8$ cycloalkyl mono- or disubstituted with $C_1$—$C_4$ alkyl, $C_1$—$C_4$ alkoxy, chloro, bromo or fluoro,

$C_1$—$C_6$ alkyl substituted with chloro, bromo or fluoro,

$C_1$—$C_6$ alkyl monosubstituted with $C_1$—$C_4$ alkoxy, $C_1$—$C_4$ alkanoyl, $C_1$—$C_4$ alkoxycarbonyl, hydroxy, amino, phenyl, or phenyl mono- or disubstituted with chloro, bromo, fluoro, nitro, cyano, $C_1$—$C_4$ alkyl, $C_1$—$C_4$ alkoxy, $C_1$—$C_4$ alkanoyl, $C_1$—$C_4$ alkoxycarbonyl, hydroxy, carboxy, amino, aminocarbonyl, mono- or di($C_1$—$C_4$ alkyl)amino, or mono- or di($C_1$—$C_4$ alkyl)aminocarbonyl;

provided that when n is 0, $R^{10}$ is not $C_1$—$C_6$ alkyl monosubstituted with phenyl monosubstituted with hydroxy and provided that carboxy, hydroxy, unsubstituted amino and unsubstituted aminocarbonyl groups are silylated with the group

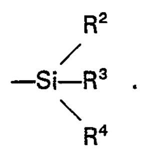

4. A silylated derivative as claimed in claim 3 wherein n is 0 and $R^{10}$ is

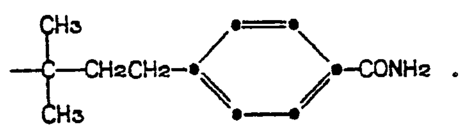

**Patentansprüche**

1. Verfahren zur Herstellung eines Amins der Formel

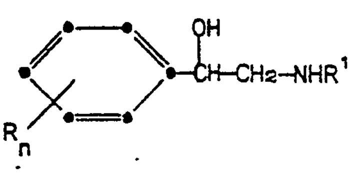

oder eines Säureadditionssalzes hiervon, worin

n für 0, 1 oder 2 steht,

R gleiche oder auch unterschiedliche Gruppen sind, falls n für 2 steht, die ausgewählt sind aus Chlor

Brom, Fluor, Nitro, $C_1$—$C_4$-Alkyl, $C_1$—$C_4$-Alkoxy, $C_1$—$C_4$-Alkylsulfonyl, Hydroxy, Carboxy, Amino, Aminocarbonyl, Mono- oder Di($C_1$—$C_4$-alkyl)amino oder Mono- oder Di($C_1$—$C_4$-alkyl)aminocarbonyl, wobei jedoch, fall n für 2 steht, die Gruppen R sich nicht beide zugleich in ortho-Stellung zur Seitenkette befinden können und wobei, falls R eine Alkoxygruppe ist, sich diese nicht in para-Stellung befinden kann, und

$R^1$ für $C_3$—$C_8$-Cycloalkyl, für durch $C_1$—$C_4$-Alkyl, $C_1$—$C_4$-Alkoxy, Chlor, Brom oder Fluor mono- oder disubstituiertes $C_3$—$C_8$-Cycloalkyl, für $C_1$—$C_6$-Alkyl, für durch Chlor, Brom oder Fluor substituiertes $C_1$—$C_6$-Alkyl oder für $C_1$—$C_6$-Alkyl steht, das monosubstituiert ist durch $C_1$—$C_4$-Alkoxy, $C_1$—$C_4$-Alkanoyl, $C_1$—$C_4$-Alkoxycarbonyl, Hydroxy, Amino, Phenyl oder mono- oder disubstituiertes Phenyl, dessen Substituenten Chlor, Brom, Fluor, Nitro, Cyano, $C_1$—$C_4$-Alkyl, $C_1$—$C_4$-Alkoxy, $C_1$—$C_4$-Alkanoyl, $C_1$—$C_4$-Alkoxycarbonyl, Hydroxy, Carboxy, Amino, Aminocarbonyl, Mono- oder Di($C_1$—$C_4$-alkyl)amino oder Mono- oder Di($C_1$—$C_4$-alkyl)aminocarbonyl sind,

dadurch gekennzeichnet, daß man ein Epoxid der Formel

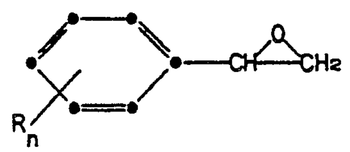

mit einem silylierten Amin der Formel

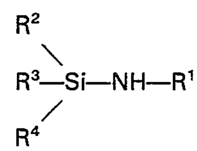

worin

$R^2$ und $R^3$ unabhängig Phenyl oder $C_1$—$C_4$-Alkyl sind und $R^4$ für $C_1$—$C_4$-Alkyl steht,

mit der Maßgabe, daß Carboxy-, Hydroxy-, unsubstituierte Amino- und unsubstituierte Aminocarbonylgruppen am Epoxid und am silylierten Amin durch die Gruppe

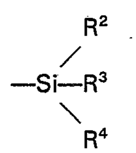

silyliert sind, in Gegenwart von Dimethylsulfoxid oder ohne ein Lösungsmittel zu einem silylierten Zwischenprodukt der Formel

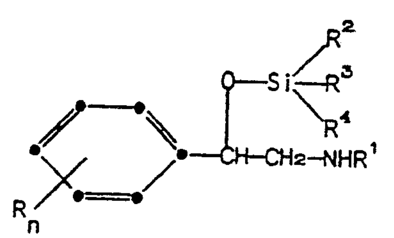

umsetzt und dieses silylierte Zwischenprodukt dann hydrolysiert.

2. Verfahren nach Anspruch 1, dadurch gekennzeichnet, daß man Verbindungen verwendet, worin n für 0 steht und $R^1$ die Formel

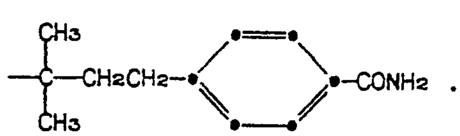

hat und so N-[3-(4-Aminocarbonylphenyl)-1,1-dimethylpropyl]-2-hydroxy-2-phenylethylamin oder ein Säureadditionssalz hiervon herstellt.

3. Silyliertes Derivat der Formel

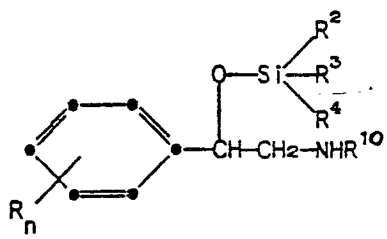

worin

n für 0, 1 oder 2 steht,

R gleiche oder auch unterschiedliche Gruppen sind, falls n für 2 steht, die ausgewählt sind aus Chlor, Brom, Fluor, Nitro, $C_1$—$C_4$-Alkyl, $C_1$—$C_4$-Alkoxy, $C_1$—$C_4$-Alkylsulfonyl, Hydroxy, Carboxy, Amino, Aminocarbonyl, Mono- oder Di($C_1$—$C_4$-alkyl)amino oder Mono- oder Di($C_1$—$C_4$-alkyl)aminocarbonyl, wobei jedoch, falls n für 2 steht, die Gruppen R sich nicht beide zugleich in ortho-Stellung zur Seitenkette befinden können, und wobei, falls R eine Alkoxygruppe ist, sich diese nicht in para-Stellung befinden kann, und

$R^{10}$ für $C_3$—$C_8$-Cycloalkyl, für durch $C_1$—$C_4$-Alkyl, $C_1$—$C_4$-Alkoxy, Chlor, Brom oder Fluor mono- oder disubstituiertes $C_3$—$C_8$-Cycloalkyl, für durch Chlor, Brom oder Fluor substituiertes $C_1$—$C_6$-Alkyl oder für $C_1$—$C_6$-Alkyl steht, das monosubstituiert ist durch $C_1$—$C_4$-Alkoxy, $C_1$—$C_4$-Alkanoyl, $C_1$—$C_4$-Alkoxycarbonyl, Hydroxy, Amino, Phenyl oder mono- oder disubstituiertes Phenyl, dessen Substituenten Chlor, Brom, Fluor, Nitro, Cyano, $C_1$—$C_4$-Alkyl, $C_1$—$C_4$-Alkoxy, $C_1$—$C_4$-Alkanoyl, $C_1$—$C_4$-Alkoxycarbonyl, Hydroxy, Carboxy, Amino, Aminocarbonyl, Mono- oder Di($C_1$—$C_4$-alkyl)amino oder Mono- oder Di($C_1$—$C_4$-alkyl)aminocarbonyl sind,

mit der Maßgabe, daß, falls n für 0 steht, $R^{10}$ nicht für $C_1$—$C_6$-Alkyl stehen kann, das durch Phenyl monosubstituiert ist, welches durch Hydroxy monosubstituiert ist, und daß die Carboxy-, Hydroxy-, unsubstituierten Amino- und unsubstituierten Aminocarbonylgruppen durch die Gruppe

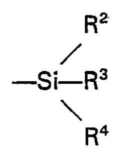

silyliert sind.

4. Silyliertes Derivat nach Anspruch 3, dadurch gekennzeichnet, daß n für 0 steht und $R^{10}$ die Formel

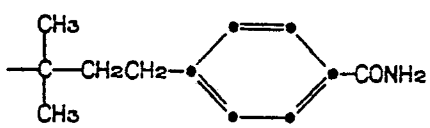 .

**Revendications**

1. Procédé de préparation d'une amine de formule:

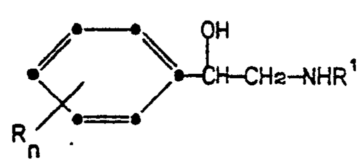

ou d'un de ses sels d'addition d'acide; où

n représente 0, 1 ou 2;

les groupes R sont identiques ou différents lorsque n est égal à 2 et ils sont choisis parmi l'atome de chlore, l'atome de brome, l'atome de fluor, le groupe nitro, les groupes alkyle en $C_1$—$C_4$, les groupes alcoxy en $C_1$—$C_4$, les groupes alkyl(en $C_1$—$C_4$)sulfonyle, le groupe hydroxy, le groupe carboxy, le groupe amino, le groupe aminocarbonyle, les groupes mono- ou di-(alkyl en $C_1$—$C_4$)amino ou les groupes mono- ou di-(alkyl en $C_1$—$C_4$)aminocarbonyle; avec cette réserve que, lorsque n est égal à 2, les groupes R ne sont pas tous deux en position *ortho* et également avec cette réserve qu'un groupe alcoxy R n'est pas en position *para*;

$R^1$ représente un groupe cycloalkyle en $C_3$—$C_8$ ou un groupe cycloalkyle en $C_3$—$C_8$ mono- ou disubstitué par un groupe alkyle en $C_1$—$C_4$, un groupe alcoxy en $C_1$—$C_4$, un atome de chlore, un atome de brome ou un atome de fluor,

un groupe alkyle en $C_1$—$C_6$,

un groupe alkyle en $C_1$—$C_6$ substitué par un atome de chlore, un atome de brome ou un atome de fluor,

un groupe alkyle en $C_1$—$C_6$ mono-substitué par un groupe alcoxy en $C_1$—$C_4$, un groupe alcanoyle en $C_1$—$C_4$, un groupe alcoxy(en $C_1$—$C_4$)carbonyle, un groupe hydroxy, un groupe amino, un groupe phényle ou un groupe phényle mono- ou disubstitué par un atome de chlore, un atome de brome, un atome de fluor, un groupe nitro, un groupe cyano, un groupe alkyle en $C_1$—$C_4$, un groupe alcoxy en $C_1$—$C_4$, un groupe alcanoyle en $C_1$—$C_4$, un groupe alcoxy(en $C_1$—$C_4$)carbonyle, un groupe hydroxy, un groupe carboxy, un groupe amino, un groupe aminocarbonyle, un groupe mono- ou di-(alkyl en $C_1$—$C_4$)amino ou un groupe mono- ou di-(alkyl en $C_1$—$C_4$)aminocarbonyle;

ce procédé consistant à faire réagir un époxyde de formule:

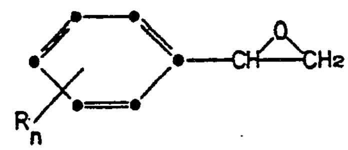

avec une amine silylée de formule:

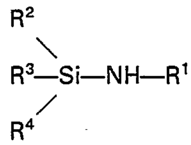

où

$R^2$ et $R^3$ représentent chacun indépendamment l'un de l'autre un groupe phényle ou un groupe alkyle en $C_1$—$C_4$, tandis que $R^4$ représente un groupe alkyle en $C_1$—$C_4$;

avec cette réserve que les groupes carboxy, hydroxy, amino non substitué et aminocarbonyle non substitué de l'époxyde et l'amine silylée sont silylés avec le groupe

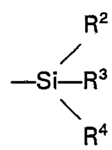 ;

en présence de diméthylsulfoxyde ou sans solvant, pour préparer un produit intermédiaire silylé de formule:

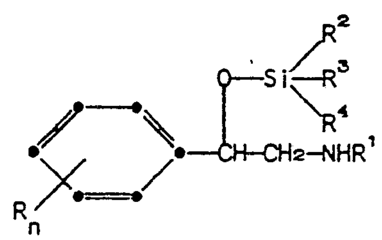

et hydrolyser le produit intermédiaire silylé.

2. Procédé selon la revendication 1, où n représente 0 et $R^1$ représente

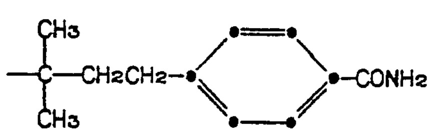 .

de façon à préparer la N-[3-(4-aminocarbonylphényl)-1,1-diméthylpropyl]-2-hydroxy-2-phényléthylamine ou un de ses sels d'addition d'acide.

3. Dérivé silylé de formule:

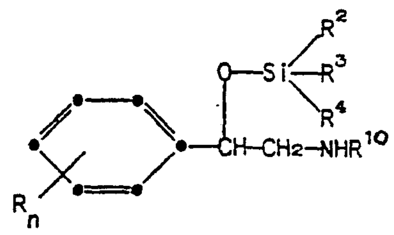

où

n représente 0, 1 ou 2;

les groupes R sont identiques ou différents lorsque n est égal à 2 et ils sont choisis parmi l'atome de chlore, l'atome de brome, l'atome de fluor, le groupe nitro, les groupes alkyle en $C_1$—$C_4$, les groupes alcoxy en $C_1$—$C_4$, les groupes alkyl(en $C_1$—$C_4$)sulfonyle, le groupe hydroxy, le groupe carboxy, le groupe amino, le groupe aminocarbonyle, les groupes mono- ou di-(alkyl en $C_1$—$C_4$)amino ou les groupes mono- ou di-(alkyl en $C_1$—$C_4$)aminocarbonyle, avec cette réserve que, lorsque n est égal à 2, les groupes R ne sont pas tous deux en position *ortho* et également avec cette réserve qu'un groupe alcoxy R n'est pas en position *para;*

$R^{10}$ représente un groupe cycloalkyle en $C_3$—$C_8$ ou un groupe cycloalkyle en $C_3$—$C_8$ mono- ou disubstitué par un groupe alkyle en $C_1$—$C_4$, un groupe alcoxy en $C_1$—$C_4$, un atome de chlore, un atome de brome ou un atome de fluor,

un groupe alkyle en $C_1$—$C_6$ substitué par un atome de chlore, un atome de brome ou un atome de fluor,

un groupe alkyle en $C_1$—$C_6$ monosubstitué par un groupe alcoxy en $C_1$—$C_4$, un groupe alcanoyle en $C_1$—$C_4$, un groupe alcoxy(en $C_1$—$C_4$)carbonyle, un groupe hydroxy, un groupe amino, un groupe phényle ou un groupe phényle mono- ou disubstitué par un atome de chlore, un atome de brome, un atome de fluor, un groupe nitro, un groupe cyano, un groupe alkyle en $C_1$—$C_4$, un groupe alcoxy en $C_1$—$C_4$, un groupe alcanoyle en $C_1$—$C_4$, un groupe alcoxy(en $C_1$—$C_4$)carbonyle, un groupe hydroxy, un groupe carboxy, un groupe amino, un groupe aminocarbonyle, un groupe mono- ou di-(alkyl en $C_1$—$C_4$)amino ou un groupe mono- ou di-(alkyl en $C_1$—$C_4$)aminocarbonyle;

avec cette réserve que, lorsque n est égal à 0, $R^{10}$ n'est pas un groupe alkyle en $C_1$—$C_6$ monosubstitué par un groupe phényle monosubstitué par un groupe hydroxy et avec cette réserve que les groupes carboxy, hydroxy, amino non substitué et aminocarbonyle non substitué sont silylés avec le groupe

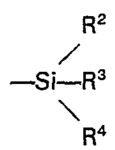

4. Dérivé silylé selon la revendication 3, dans lequel n est égal à 0 et $R^{10}$ répond à la formule: